# EUROPEAN PATENT SPECIFICATION

(11) **EP 4 236 783 B1**
(45) Date of publication and mention of the grant of the patent: **22.01.2025**
(21) Application number: 21789774.3
(22) Date of filing: 19.10.2021
(51) Int. Cl.: A61B 5/055, A61B 5/372, A61B 5/00

(54) **SYSTEM AND COMPUTER PROGRAM PRODUCT FOR ANALYZING BRAIN ACTIVITY**
SYSTEM UND COMPUTERPROGRAMMPRODUKT ZUR ANALYSE DER GEHIRNAKTIVITÄT
SYSTÈME ET PRODUIT PROGRAMME D'ORDINATEUR D'ANALYSE DE L'ACTIVITÉ CÉRÉBRALE

(30) Priority: 27.10.2020 EP 20204022
(43) Date of publication of application: 06.09.2023
(73) Proprietor: Koninklijke Philips N.V., 5656 AG Eindhoven (NL)
(72) Inventor: VAN EE, Raymond, 5656 AE Eindhoven (NL); WESTERINK, Joanne Henriëtte Desirée Monique, 5656 AE Eindhoven (NL); LEUFKENS, Timmy Robertus Maria, 5656 AE Eindhoven (NL); MENA BENITO, Maria Estrella, 5656 AE Eindhoven (NL); DENISSEN, Adrianus Johannes Maria, 5656 AE Eindhoven (NL); HUIJBERS, Willem, 5656 AE Eindhoven (NL)
(74) Representative: Philips Intellectual Property & Standards
(86) International application number: PCT/EP2021/078881
(87) International publication number: WO 2022/089987

(56) References cited:
- EP-A1- 3 669 922
- EP-A2- 1 742 155
- JP-A- 2019 195 469
- LONG XI ET AL: "Time delay between cardiac and brain activity during sleep transitions", APPLIED PHYSICS LETTERS, AMERICAN INSTITUTE OF PHYSICS, 2 HUNTINGTON QUADRANGLE, MELVILLE, NY 11747, vol. 106, no. 14, 6 April 2015 (2015-04-06), XP012196355, ISSN: 0003-6951, [retrieved on 19010101], DOI: 10.1063/1.4917221
- TAKAHASHI K. ET AL: "Neuronal Activity of Histaminergic Tuberomammillary Neurons During Wake-Sleep States in the Mouse", THE JOURNAL OF NEUROSCIENCE, vol. 26, no. 40, 4 October 2006 (2006-10-04), US, pages 10292 - 10298, XP009532868, ISSN: 0270-6474, DOI: 10.1523/JNEUROSCI.2341-06.2006
- ANDRADE PEDRO ET AL: "Generalized Seizures after Experimental Traumatic Brain Injury Occur at the Transition from Slow-Wave to Rapid Eye Movement Sleep", vol. 34, no. 7, 1 April 2017 (2017-04-01), US, pages 1482 - 1487, XP055792219, ISSN: 0897-7151, Retrieved from the Internet <URL:http://dx.doi.org/10.1089/neu.2016.4675> DOI: 10.1089/neu.2016.4675
- TSUNO N ET AL: "Fluctuations of source locations of EEG activity during transition from alertness to sleep in Alzheimer's disease and vascular dementia", NEUROPSYCHOBIOLOGY, vol. 50, no. 3, 2004, pages 267 - 272, XP009526714, ISSN: 0302-282X
- KISHI AKIFUMI ET AL: "Dynamics of sleep stage transitions in healthy humans and patients with chronic fatigue syndrome", AMERICAN JOURNAL OF PHYSIOLOGY-REGULATORY , INTEGRATIVE AND COMPARATIVE PHYSIOLOGY, vol. 294, no. 6, 1 June 2008 (2008-06-01), US, pages R1980 - R1987, XP055792120, ISSN: 0363-6119, DOI: 10.1152/ajpregu.00925.2007
- BURNETT K. F, TAYLOR C. B, THORESEN C. E, ROSEKIND M. R, MILES L. E, DE BUSK R. F: "Toward computerized scoring of sleep using ambulatory recordings of heart rate and physical activity", BEHAVIORAL ASSESSMENT, vol. 7, no. 3, 1 January 1985 (1985-01-01), pages 261 - 271, XP002805410, ISSN: 0191-5401

## Description

### FIELD OF THE INVENTION

The invention relates to the field of analyzing the brain activity of a subject, and in particular to analyzing a subject's brain activity during a transition between brain states.

### BACKGROUND OF THE INVENTION

At present, the diagnosis of mental disorders is based on a combination of psychiatric interview, questionnaires and patients' own accounts. The diagnosis of a patient is therefore somewhat subjective. The accounts and responses given by a patient are colored by the patient's views and perceptions, and the interpretation of these accounts and responses into a diagnosis depend, to an extent, on the views and perceptions of the clinician making the diagnosis.

Further, some patients may be unable to provide the clinician with the necessary information for a diagnosis as a result of their mental disorder(s). Some mental disorders will affect a patient's memory, and patients with anxiety may find interviews with a clinician stressful, leading them to omit important information. In some cases, the clinician may rely on information from a third party, but this may provide only a partial picture, and, again, the information is subjective.

There is therefore a need for an objective diagnostic tool for diagnosing mental disorders accurately and reliably.

Takahashi et al. 2006, "Neuronal activity of histaminergic tuberomammillary neurons during wake-sleep states in the mouse", The Journal of Neuroscience, 26(40):10292-10298 discloses monitoring of neuronal activity of histaminergic tuberomammillary neurons during wake-sleep states in mice.

Andrade et al. 2017, "Generalized seizures after experimental traumatic brain injury occur at the transition from slow-wave to rapid eye movement sleep", Journal of Neurotrauma, 34(7):1482-1487 discloses monitoring a transition from stage III to rapid eye movement sleep in rats with generalized seizures.

Tsuno et al. 2004, "Fluctuations of source locations of EEG activity during transition from alertness to sleep in Alzheimer's disease and vascular dementia", Neuropsychobiology, 50(3):267-272 discloses monitoring a transition from alertness to sleep in Alzheimer's disease and vascular dementia.

JP 2019 195469 A discloses a sleep evaluation method.

EP 1,742,155 A2 discloses a method for determining a clinical state of a subject.

EP 3,669,922 A1 discloses a system and method that adapts how an audio output is generated based on a response of a subject's sleep parameters to the audio output.

Kishi et al. 2008, "Dynamics of sleep stage transitions in healthy humans and patients with chronic fatigue syndrome", American Journal of Physiology-Regulatory, Integrative and Comparative Physiology, 294(6):R1980-R1987 discloses studying dynamics of sleep stage transitions in healthy humans and patients with chronic fatigue syndrome.

Burnett et al. 1985, "Toward computerized scoring of sleep using ambulatory recordings of heart rate and physical activity", Behavioral Assessment, 7(3):261-271 discloses a comparison of an ambulatory monitoring system for recording heart rate and physical activity level with polysomnography.

Long Xi et al. 2015: "Time delay between cardiac and brain activity during sleep transitions", Applied Physics Letters, vol. 106, no. 14, 6 April 2015, describes an investigation of the time delay between changes of cardiac and brain activity for sleep transitions. The brain activity was quantified by electroencephalographic (EEG) mean frequency and the cardiac parameters included heart rate, standard deviation of heartbeat intervals, and their low- and high-frequency spectral powers.

### SUMMARY OF THE INVENTION

The invention is defined by the independent claims. Advantageous embodiments are defined in the dependent claims.

According to examples in accordance with an aspect of the invention, there is provided a processing system for analyzing the brain activity of a subject during a transition between brain states.

The processing system is configured to: receive, from a brain monitoring system for monitoring brain activity, brain activity data of the subject obtained during a transition of the subject from a first brain state to a second brain state of the subject, wherein at least one of the first brain state and/or the second brain state is a sleep state; receive, from a sensory and monitoring unit adapted to detect a transition of the subject from the first brain state to the second brain state of the subject, information corresponding to the detected transition and process the brain activity data and the information from the sensory and monitoring unit to obtain a value for one or more transition parameters of the brain activity data, a transition parameter being a parameter representative of the transition.

The one or more transition parameters comprise at least one of: a transition timing, a transition duration, a transition stability and/or a frequency of transition. A transition timing is a length of time between a time at which a transition is detected in the subject and a time at which changes in neuronal networks, identifiable in the subject's brain activity data, responsive to the change in sleep state are first detected. A transition duration is a duration from a moment at which a neuronal network associated with the first brain state of the subject starts to become weaker to a moment at which a neuronal network associated with the second brain state becomes fully established. A transition stability is a number of transitions between neuronal networks in the brain activity data from a moment at which activity in neuronal networks associated with the first brain state starts to become weaker to a moment at which a network associated with the second brain state becomes fully established. A frequency of transition is a measure of the number of times a transition from the first brain state to the second brain state occurs in a set time period.

This system may be used to support diagnosis of mental disorders. The inventors have recognized that brain activity is different in different states. For example, resting state wakeful activity is different to brain activity during sleep.

The inventors have further recognized that psychiatric patients generally exhibit different, weakened or delayed transitions between different brain states. Thus, parameters derived from monitoring the brain activity of a subject during a transition can be used as a biomarker for assessing or identifying any mental disorders of the patient.

The processing system may be further configured to display the obtained values of the parameters. This provides a clinician with useful clinical information for assessing the condition and/or state of the subject, and in particular, to aid in the assessment of whether or not the subject is associated with a mental disorder and/or condition.

The brain monitoring system may be a magnetic resonance imaging (MRI) scanner. However, it will be appreciated that the brain monitoring system may instead or additionally employ other brain scanning techniques such as an electroencephalography (EEG) methodology and/or a positron emission tomography (PET) technique. In some embodiments, the brain monitoring system may employ a combination of different brain scanning techniques, e.g. a combination of EEG and MRI.

Thus, the brain activity data may be MRI data, EEG data, PET data or any combination of the same. Of course, the type of brain activity data will depend upon the type of brain monitoring system used.

The processing system is further configured to process the values of the one or more transition parameters of the brain activity data and corresponding values of the one or more transition parameters for a plurality of groups of subjects to identify which of the plurality of groups the subject most closely resembles, wherein the plurality of groups of subjects comprises at least a first group and a second group, wherein the first group comprises healthy subjects and the second group comprises subjects having a mental disorder.

In this way, the brain activity of the subject during a transition may be compared with that of healthy subjects and subjects with mental disorders in order to determine which the subject's brain activity is most like. In particular, the subjects (in the plurality of groups of subject) may be grouped based on at least a mental disorder (or absence thereof) in each subject. Thus, each group of subjects may represent a group of subjects sharing a same (particular) mental disorder and/or lack of a (particular) mental disorder.

The step of processing the values of the one or more transition parameters of the brain activity data and corresponding values of the one or more transition parameters for a plurality of groups of subjects further uses one or more characteristics of the subject to identify which of the plurality of groups the subject most closely resembles.

The transition experienced by the subject may depend on factors other than those used to group the plurality of groups of subjects. For example, the subjects may be grouped based on mental disorders, but the transition experienced by the subject may also be affected by sleep medication use. Accounting for these factors reduces the likelihood of providing information that would cause a healthy subject to be misdiagnosed with a mental disorder or a subject with a mental disorder to be incorrectly diagnosed as healthy.

The one or more characteristics of the subject may comprise at least one of: a sleep medication use of the subject, age, sex, left- or right-handedness, previous sleep quality and/or chronotype. It has been recognised that each of these parameters can also have an influence or effect on the transition of the patient between sleep states. It would therefore be advantageous to take such characteristics into account when assessing a psychiatric state of the patient.

For example, sleep medication may cause a subject to fall asleep faster. Taking this into account reduces the likelihood that the group the subject's brain activity is determined to most closely resemble is unaffected by any sleep medication taken by the subject.

The one or more characteristics may be accounted for by subdividing each of the plurality of groups of subjects according to the one or more characteristics. The one or more transition parameters of the brain activity data and corresponding values of the one or more transition parameters for the subdivided groups with the same one or more characteristics as the subject may be processed to identify which of the subdivided groups the subject most closely resembles.

In some embodiments, the step of processing the values of the one or more transition parameters of the brain activity data and corresponding values of the one or more transition parameters for a plurality of groups of subjects to identify which of the plurality of groups the subject most closely resembles comprises inputting the brain activity data and/or the values of the one or more transition parameters into an artificial neural network.

The artificial neural network may be trained using a training algorithm configured to receive an array of training inputs and known outputs, wherein the training inputs comprise brain activity data and/or values of one or more transition parameters during transitions from a first brain state to a second brain state, and the known outputs comprise a determination of which of a plurality of groups of subjects the brain activity data belongs to. In other words, the artificial neural network may classify the brain activity data.

In this way, an artificial neural network may be trained to identify features in the transitions that are representative of a particular group of subjects and that may be used to distinguish between different groups. The groups of subjects, include a group of healthy subjects and one or more groups of subjects with mental disorders.

The transition may be one of: a transition from a wakeful state to a sleep state; a transition from a sleep state to a wakeful state; or a transition from a first sleep state to a second, different sleep state.

Different neuronal networks are involved in different sleep states and wakeful states, so transitions between a wakeful and a sleep state and transitions between different sleep states may be used to obtain transition parameters in the brain activity data.

The one or more transition parameters may comprise at least one of: a transition timing, a transition duration, a transition stability, a frequency of transition and/or the one or more networks active during the transition.

A transition timing may be defined as the time between the moment of the detected transition and the moment the neuronal network associated with the first brain state of the subject starts to become weaker in the brain activity imaging data.

A transition duration may be defined as the duration from the moment the neuronal network associated with the first brain state of the subject starts to become weaker until the neuronal network associated with the second brain state of the subject is fully established.

A transition (in)stability may be defined as the number of transitions between various neuronal networks during the time between the moment the neuronal network associated with the first brain state of the subject starts to become weaker and the moment the neuronal network associated with the second brain state of the subject is fully established.

A frequency of transition may be defined as the number of times a transition between different brain states occurs within a particular time period.

Subjects with mental disorders may be expected to have a delayed transition timing, a longer transition duration, a higher transition instability, more frequent transitions and different active neuronal networks during the transition compared to healthy subjects. The particular differences in these parameters exhibited by a subject will depend on the particular mental disorder(s) the subject has.

The plurality of groups of subjects may comprise at least a first group and a second group, wherein the first group comprises healthy subjects and the second group comprises subjects having a (particular) mental disorder. In this way, the system may be used to determine whether the brain activity of the subject more closely resembles a healthy subject or a subject with a (particular) mental disorder.

In some embodiments, the processing system is configured to continue receiving brain activity data of the subject until a predefined number of transitions have been recorded, and to obtain a value for one or more transition parameters of the brain activity data for each detected transition.

This may improve the reliability of the obtained value(s) of the one or more parameters.

There is also proposed a system comprising: a sensory and monitoring unit adapted to detect a transition of the subject from a first brain state to a second brain state of the subject, wherein at least one of the first brain state and/or the second brain state is a sleep state; and the processing system described above, further configured to receive, from the sensory and monitoring unit, information corresponding to the detected transition.

The sensory and monitoring unit may be used to accurately detect the subject's transition between brain states. Information corresponding to the detected transition may, for example, comprise a time at which the transition was detected.

The sensory and monitoring unit may be adapted to detect a transition based on at least one of: brain activity information, cardiorespiratory information, cardioballistography information, respiration rate, behavioral information and/or information corresponding to the subject's performance on a repetitive task.

The sensory and monitoring unit may obtain brain activity information from the brain monitoring system, or may directly image the ventrolateral preoptic nucleus of the hypothalamus. Cardiorespiratory information may be obtained from a PPG sensor or a vital signs camera. Respiration rate may be measured using radar technology. Behavioral information may be obtained from a vital signs camera. Information corresponding to the subject's performance on a repetitive task may include reaction time and/or percentage correct answers.

In some embodiments, the system further comprises a sleep regulatory unit adapted to induce a change in the brain state of the subject.

The sleep regulatory unit may be adapted to induce sleep in the subject, wake the subject from sleep or induce a change from a first sleep state to a second, different sleep state. Inducing a change in brain state of the subject ensures that the subject undergoes the transition being recorded promptly. If a subject is left to fall asleep, wake up or change from one sleep state to another without assistance, this may take a long time. Use of a sleep regulatory unit therefore provides a more controlled environment for obtaining values for the transition parameter(s) for the patient.

The sleep regulatory unit may induce sleep by, for example, the use of tactile feedback and/or the generation of a rhythmic sound. The sleep regulatory unit may be adapted to induce sleep by decreasing the breathing rate of the subject.

In some embodiments, the sleep regulatory unit is adapted to alternately induce sleep in the subject and wake the subject from sleep for a predetermined number of wake/sleep cycles.

In this way, a number of transitions may be recorded efficiently. The sleep regulatory unit may be adapted to wake a sleeping subject once the neuronal network associated with a sleep state has been fully established, and to induce sleep in a wakeful subject once the neuronal network associated with a wakeful state has been fully established.

There is also proposed a computer program product comprising computer program code which, when executed on a computing device having a processing system, cause the processing system to perform a computer-implemented method for analyzing the brain activity of a subject during a transition between brain states.

The computer-implemented method comprises: receiving, from a brain monitoring system for monitoring brain activity, brain activity data of the subject obtained during a transition of the subject from a first brain state to a second brain state of the subject, wherein at least one of the first brain state and/or the second brain state is a sleep state; receiving, from a sensory and monitoring unit adapted to detect a transition of the subject from the first brain state to the second brain state of the subject, information corresponding to the detected transition; processing the brain activity data and the information from the sensory and monitoring unit to obtain a value for one or more transition parameters of the brain activity data, a transition parameter being a parameter representative of the detected transition, one or more transition parameters comprise at least one of: a transition timing, a transition duration, a transition stability and/or a frequency of transition, as defined above; and processing the values of the one or more transition parameters of the brain activity data and corresponding values of the one or more transition parameters for a plurality of groups of subjects to identify which of the plurality of groups the subject most closely resembles, wherein the plurality of groups of subjects comprises at least a first group and a second group, wherein the first group comprises healthy subjects and the second group comprises subjects having a mental disorder.

These and other aspects of the invention will be apparent from and elucidated with reference to the embodiments described hereinafter.

### BRIEF DESCRIPTION OF THE DRAWINGS

Examples of the invention will now be described in detail with reference to the accompanying drawings, in which:
Fig. 1 shows a system for analyzing the brain activity of a subject during a transition between brain states, according to an embodiment of the invention; and
Fig. 2 shows a method for analyzing the brain activity of a subject during a transition between brain states, as implemented by a system and a computer program product according to an embodiment of the invention.

### DETAILED DESCRIPTION OF THE EMBODIMENTS

The invention will be described with reference to the Figures.

It should be understood that the detailed description and specific examples, while indicating exemplary embodiments of the invention, are intended for purposes of illustration only and are not intended to limit the scope of the invention. These and other features, aspects, and advantages of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and computer program product for analyzing the brain activity of a subject during a transition between brain states. Brain activity data including data representative of a transition between brain states is received from a brain monitoring system and processed to obtain a value for one or more transition parameters. The one or more transition parameters are parameters that characterize the transition between brain states and may be used as a biomarker for identifying mental disorders.

Embodiments are at least partly based on the realization that the transitions between brain states are different, weakened or delayed in subjects with mental disorders when compared with healthy subjects. Parameters representative of transitions between brain states may therefore act as biomarkers for mental disorders, enabling a more precise and reliable diagnosis of mental disorders.

Illustrative embodiments may, for example, by employed in clinical decision support systems.

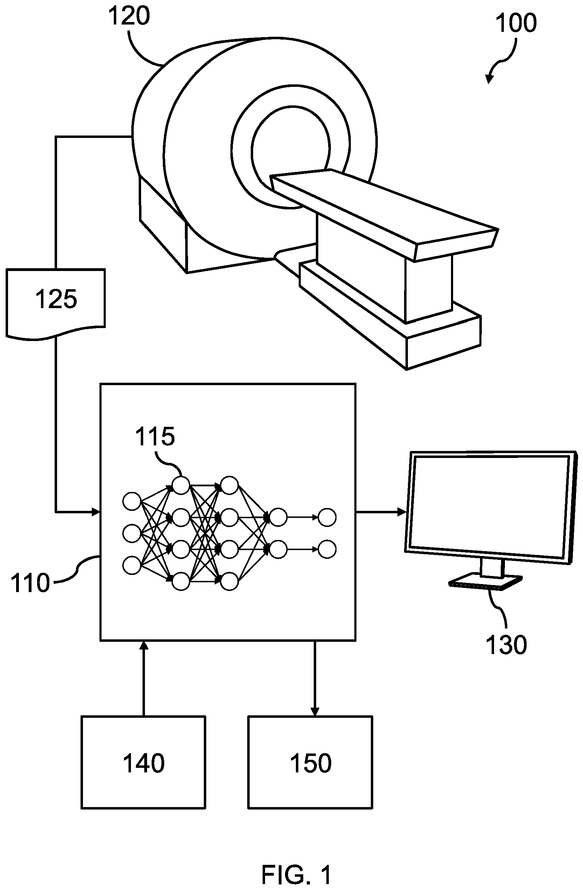

Fig. 1 illustrates a system 100 for analyzing the brain activity of a subject during a transition between brain states, comprising a processing system 110, according to an embodiment of the invention. The processing system 110 is, itself, an embodiment of the invention.

The processing system 110 is in communication with a brain monitoring system 120. In Fig. 1, the brain monitoring system 120 is an MRI scanner. MRI scanners are frequently used for making the activity in neuronal networks visible, but any brain monitoring system suitable for monitoring brain activity may be used. For example, the brain monitoring system may be a PET scanner or EEG machine. The brain monitoring system may comprise more than one brain monitoring apparatus. For example, the brain monitoring system may comprise an MRI scanner and an EEG machine.

The brain monitoring system 120 obtains brain activity data 125 of a subject during a time including at least one transition from a first brain state of the subject to a second brain state. At least one of the first and second brain states is a sleep state, that is, a brain state corresponding to a sleep stage of the subject, for example, stage 1 sleep, stage 2 sleep, slow wave sleep or REM sleep. For example, the transition may be a transition from a wakeful state to a sleep state, a transition from a sleep state to a wakeful state, or a transition from a first sleep state to a second, different sleep state.

The brain activity data 125 comprises information indicative of the activity of a plurality of neuronal networks over a time including the at least one transition between brain states. Different neuronal networks are active in different brain states, so the activity of the neuronal networks can be used to monitor a transition between brain states.

The processing system 110 receives the brain activity data 125 from the brain monitoring system 120, and processes the brain activity data to obtain a value for one or more transition parameters of the brain activity data. A transition parameter is a parameter representative of the transition between brain states. Subjects with mental disorders exhibit different transitions between brain states to healthy subjects, so parameters representative of transitions between brain states will be different for healthy subjects and subjects with mental disorders.

The brain activity data can indicate a sleep state of the subject. In particular, a subject has different neural activity levels depending upon their sleep state. It is therefore possible to detect a current sleep state of the subject, as well as to detect a transition between one sleep state and another. Mechanisms for detecting a sleep state and/or a transition in sleep states are well known to the skilled person, e.g. as set out in the International Patent Applications having publication numbers WO 2016/193030 A1, WO 2015/118415 A1 or WO 2013/061185 A1.

The processing system 120 may use further parameters of the patient (e.g. other physiological signals) to derive the transition parameters. Thus, the processing system 120 may employ a receive information from a sensory and monitoring unit 140. The unit 140 may be usable to detect a physical indication of a change in transition state (e.g. change in heartrate, respiratory rate of the like), and the brain activity data may be usable to detect a neurological indication of a change in sleep state (e.g. when the active neuronal network(s) changes).

The one or more transition parameters may, for example, include a transition timing, a transition duration, a transition stability, a frequency of transition and/or the one or more networks active during the transition. Other suitable transition parameters will be apparent to the skilled person.

A transition timing is the length of time between the time at which a transition is (first) detected in the subject (e.g. by a sensory and monitoring unit 140) and the time at which changes in the neuronal networks, identifiable in the subject's brain activity data 125, responsive to the change in sleep state are first detected. In other words, a transition timing may be the delay between a time at which sleep state of the subject begins to change (as indicated by physiological signals) and the time at which the brain activity data first shows a change in the activity of neuronal network(s). A change in the neuronal network may, for instance, be indicated by a neuronal network associated with the first brain state starting to become weaker.

Various methods for detecting the transition in the subject are envisaged, and are described in more detail below. The start of the transition in the brain activity data may be considered to be the moment at which activity in the neuronal networks associated with the first brain state starts to become weaker.

Subjects with mental disorders tend to experience delayed transitions between brain states when compared with healthy subjects, so a long transition timing may be indicative of a mental disorder. A transition timing of the order of tens of seconds may be considered to be a long transition timing, e.g. a transition timing greater than 10s, e.g. more than 50s.

A transition duration is the length of time the transition takes in the brain activity data 125. This may be measured as the time from the moment at which activity in the neuronal networks associated with the first brain state starts to become weaker to the moment at which the network associated with the second brain state becomes fully established.

The precise mechanism by which a "weaker" and "fully established" neural network are detected may depend upon the form of the brain activity data.

Consider a scenario in which the brain activity data is represented by a stream of (2D/3D) images, such as MR images. The strength of a neuronal network may be defined by the number of voxels (representing a particular neural network) that display as active in the brain monitoring data, e.g. the number of voxels that identify ongoing brain activity within a particular neural network. A neuronal network may be considered to become weaker when the (average) number of voxels associated with the neuronal network that display as active decreases by more than a predetermined amount and/or percentage, or drop below some predetermined threshold. A neuronal network may be considered fully established when the number of voxels, associated with the neuronal network, that display as active exceeds some predefined threshold.

In another example, the brain activity data may comprise EEG data, and a neuronal network may be considered active if a particular combination of signals (e.g. that represent activity in the neuronal network) meet some predetermined criteria.

A transition between brain states may be expected to take longer in subjects with mental disorders than in healthy subjects. A long transition duration may therefore suggest that a subject has a mental disorder.

A transition stability is a measure of how many transitions between neuronal networks occur in the course of the transition from the first brain state to the second brain state. For example, transition stability may be measured as the number of transitions between neuronal networks in the brain activity data 125 during the time from the moment at which activity in the neuronal networks associated with the first brain state starts to become weaker to the moment at which the network associated with the second brain state becomes fully established.

Subjects with mental disorders generally exhibit less stable transitions between brain states than healthy subjects. A high number of transitions between neuronal networks during a transition between brain states may therefore indicate that a subject has a (particular) mental disorder.

A frequency of transition is a measure of the number of times a given transition occurs in a set time period. For example, in the case of a wake to sleep transition, the number of times that sleep onset is detected in the brain activity data 125 within a certain time period, for example, within a five minute period.

The particular neuronal networks that are active during the transition between brain states may also be used as a biomarker for mental disorders. In particular, it is believed that the brain networks that are active during a transition between sleep states will differ between patients having a particular mental disorder and patient not having the same mental disorder. For instance, if a subject's brain activity data shows that a neuronal network not typically associated with a particular type of transition is active during that transition for that subject, this may be used to indicate that the subject has a mental disorder.

In some embodiments, the system 100 may further comprise a display device 130 in communication with the processing system 110. The display device 130 may receive, from the processing system 110, and display the obtained value(s) of the one or more transition parameters. A clinician may use the displayed value(s) in order to aid the diagnosis of the subject with a mental disorder, for example, by comparing the displayed value(s) with reference values.

Alternatively, or additionally, the processing system 110 may process the obtained value(s) of the one or more transition parameters to determine whether the obtained value(s) indicate that the subject is healthy or has a mental disorder. The processing system may also determine which one or more mental disorders are indicated by the obtained value(s).

The processing system 110 may determine what the obtained value(s) indicate about the mental health of the subject by processing the obtained value(s) and corresponding values of the one or more transition parameters for a plurality of groups of subjects in order to determine which of the plurality of groups the obtained value(s) for the subject most closely resembles. The processing system may send an indication of which group the subject most closely resembles to the display device 130.

For example, the processing system 110 may compare the obtained value(s) of the one or more transition parameters for the subject with corresponding values for a group of healthy subjects and corresponding values for a group of subjects with mental disorders and determine whether the obtained value(s) more closely resemble the corresponding values for the group of healthy subjects or the corresponding values for the group of subjects with mental disorders. In this way, the processing system may determine whether the obtained value(s) indicate(s) that the subject is healthy or has a mental disorder.

The processing system 110 may compare the obtained value(s) for the subject with corresponding values for a group of healthy subjects and for a plurality of groups of subjects with mental disorders, where each group is composed of subjects with a different mental disorder. For example, the obtained value(s) for the subject may be compared with corresponding values for a group of subjects with a first mental disorder and corresponding values for a group of subjects with a second, different mental disorder. The plurality of groups of subjects may also comprise groups of subjects with particular combinations of two or more mental disorders. In this way, the processing system may determine which mental disorder (or mental disorders) is indicated by the obtained value(s).

In some embodiments, the processing system 110 may identify which of a plurality of groups the subject most closely resembles by using a lookup table comprising values of transition parameters and the group of subjects the values correspond to.

In some embodiments, the processing system 110 may provide the brain activity data and/or the value(s) of the transition parameter(s) to an artificial neural network 115 in order to identify which of a plurality of groups the subject most closely resembles.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, (weightings of) the mathematical operation of each neuron may be modified until the error converges. Known methods of modifying a neural network include gradient descent, backpropagation algorithms and so on.

The training input data entries for the artificial neural network 115 correspond to example brain activity data and/or value(s) of transition parameter(s) during transitions from a first brain state to a second brain state. The training output data entries correspond to an identity of which of a plurality of groups of subjects the brain activity data belongs to.

Other methods of determining which of a plurality of groups a subject most closely resembles based on value(s) of one or more transition parameters will be apparent to the skilled person.

In some embodiments, the processing system may be adapted to take into account one or more characteristics of the subject that may affect the transition between brain states experienced by the subject. Characteristics that may affect transitions between brain states will be apparent to the skilled person and may include sleep medication use (or other medication use), age, sex, left- or right-handedness, previous sleep quality and/or chronotype.

For example, each of the plurality of groups of subjects may be further subdivided according to one or more characteristics. The obtained value(s) for the one or more transition parameters for the subject may then be compared with corresponding values for the subdivided groups comprised of subjects with the same one or more characteristics. For example, for a subject taking a particular medication, the obtained value(s) for the one or more transition parameters may be compared with corresponding values for a group of healthy subjects that take the same medication as the subject, for a group of subjects with a first mental disorder that take the same medication, for a group of subjects with a second mental disorder that take the same medication, etc. In this way, the determination of whether the subject most closely resembles a healthy subject, a subject with the first mental disorder, a subject with the second mental disorder etc. is less likely to be affected by the effect that the medication has on the transitions between brain states experience by the subject.

In some embodiments, the system 100 is configured to obtain and process brain activity data 125 over a plurality of transitions between brain states. For example, the processing system 110 may be configured to continue receiving brain activity data from the brain monitoring system 120 until a predefined number of transitions have been recorded. The predefined number of transitions to record may be a predefined number of a particular type of transition. For example, the processing system may be configured to continue receiving brain activity data until a predefined number of transitions from a wakeful state to a sleep state has been recorded. Alternatively, the predefined number of transitions to record may be a predefined number of various different transitions. For example, the processing system may be configured to continue receiving brain activity data until a predefined number of each of a plurality of transition types, or a predefined number of total transitions, have been recorded.

Methods of choosing a suitable predefined number of transitions to record will be apparent to the skilled person. For example, the predefined number may be determined using a power analysis to find a number of transitions that can obtain significant effects.

The processing system 110 may obtain a value for one or more transition parameters for each of the plurality of transitions. The processing system may determine an average value (e.g. a median, mean or modal value) for each of the one or more transition parameters based on the obtained values. The processing system may compare the average value(s) with corresponding values for a plurality of groups of subjects in order to determine which group the subject most closely resembles.

In some embodiments, the system 100 may further comprise a sensory and monitoring unit 140. The sensory and monitoring unit detects transitions between a first brain state and second brain state of the subject. For example, the sensory and monitoring unit may detect when the subject falls asleep, when the subject wakes up, and/or when the subject experiences a transition from one sleep stage to another sleep stage.

Methods for detecting transitions between brain states will be apparent to the skilled person. For example, the sensory and monitoring unit 140 may detect a transition based on brain activity data. This may be the brain activity data 125 obtained by the brain monitoring system 120, or brain activity data obtained from another brain monitoring apparatus. The brain activity data used to detect a transition may, for example, comprise imaging data of the ventrolateral preoptic nucleus of the hypothalamus of the subject. The sensory and monitoring unit may detect a transition between brain states based on cardiorespiratory information, for example, by using a PPG sensor and/or a vital signs camera. The sensory and monitoring unit may detect a transition between brain states based on cardioballistography information. The sensory and monitoring unit may detect a transition between brain states based on respiration rate. Respiration rate may, for example, be measured using radar technology. The sensory and monitoring unit may detect a transition based behavioral information, such as eye movement, eye blinks, alertness measures, reaction times and response to stimuli. Behavioral information may, for example, be obtained using a vital signs camera. In the case of transitions between wakeful and sleep states, the sensory and monitoring unit may detect a transition using indirect methods, such as measuring information corresponding to the subject's performance on a repetitive task. For example, the sleep and monitoring unit may monitor the subject's reaction time or percentage correct answers while the subject carries out a repetitive task.

The sensory and monitoring unit 140 is in communication with the processing system 110. The processing system receives from the sensory and monitoring unit information corresponding to transitions detected by the sensory and monitoring unit. The information may, for example, comprise a time at which a transition was detected by the sensory and monitoring unit. The information may further comprise additional information about the detected transition, such as a transition type. The time at which a transition was detected by the sensory and monitoring unit may be used by the processing system to determine a transition timing for the detected transition.

As previously explained, the processing system 110 may further use information from the sensory and monitoring unit 140 to determine values for one or more transition parameters. Thus, the step of processing the brain activity data to determine values for one or more transition parameters may comprise processing the brain activity data and the information from the sensory and monitoring unit to determine values for one or more transition parameters.

For instance, one of the transition parameter(s) may be a transition timing, being a different between a sleep state transition as indicated by the sensory and monitoring unit 140 and a change in neuronal networks (from the brain activity data) indicating a transition between sleep states. A delay between a detected sleep state transition and a corresponding transition in neuronal state may be indicative of a mental disorder.

In some embodiments, transitions between brain states in the subject may occur spontaneously. For example, if the subject is undergoing a brain scan without being engaged in a sensory and/or cognitive task, the subject may spontaneously fall asleep.

In other embodiments, the system 100 may further comprise a sleep regulatory unit 150 adapted to induce a change in brain state of the subject. For example, the sleep regulatory unit may induce sleep in the subject, wake the subject from sleep and/or induce a change in the subject from a first sleep state to a second, different sleep state.

Methods of inducing a change in brain states will be apparent to the skilled person and may depend on the type of transition being induced. For example, the sleep regulatory unit 150 may induce sleep using tactile feedback, such as the use of a breathing balloon in the hand or on the chest of the subject. The subject may be asked to breathe in as the breathing balloon expands and out as the breathing balloon contracts. Finger PPG may be used to measure the breathing rate of the subject and the measured breathing rate fed to the breathing balloon. The breathing balloon may start expanding and contracting at the current breathing rate of the subject and slowly decrease the subject's breathing rate via a feedback loop of the finger PPG signal. A decrease in breathing rate will generally induce relaxation in the subject and increase the likelihood of the subject's falling asleep. The sleep regulatory unit may use sound to induce sleep in the subject, for example, by generating a rhythmic ticking sound. The sleep regulatory unit may wake a subject from sleep by the use of, for example, sound, tactile feedback and/or changes in light levels. The sleep regulatory unit may induce a change in the subject from a first sleep state to a second, different sleep state by the use of sensory stimulation such as light, or gentle auditory or tactile signals.

Other mechanisms for facilitating sleep state transitions may employ techniques such as those suggested by the International Patent Applications having publication numbers WO 2015/087188 A1 or WO 2018/104309 A1.

In some embodiments, the sleep regulatory unit 150 alternately induces sleep in the subject and wakes the subject from sleep. The sleep regulatory unit may continue to induce sleep and wakefulness for a predetermined number of wake/sleep cycles to allow a plurality of transitions between brain states to be monitored. The predetermined number of wake/sleep cycles may be equal to the predefined number of transitions recorded by the system 100 described above.

When the sleep regulatory unit 150 induces a plurality of transitions between brain states in the subject, it is desirable that sufficient time is left between the induced transitions for a first transition to be completed before a second transition is induced. For example, if the sleep regulatory unit induces sleep in a subject and then wakes the subject from sleep, it may be desirable that the sleep regulatory unit does not begin to wake the subject until the neuronal networks associated with the sleep state have been fully established in the patient, in order that the brain activity data associated with the wake to sleep transition includes all the information necessary for the processing system 110 to obtain a value for one or more transition parameters.

The sleep regulatory unit 150 may, for example, be configured to induce transitions at predetermined time intervals, for example, every 5 minutes, every 10 minutes or every 20 minutes. The predetermined time interval may be no less than 5 minutes, e.g. no less than 10 minutes. Alternatively, the sleep regulatory unit may be configured to induce a transition on an instruction from the processing system 110. For example, the processing unit may determine, based on the brain activity data 125 and/or information from the sensory and monitoring unit 140, that a sleep network has been fully established in the subject, and send an instruction to the sleep regulatory unit to wake the subject up.

In an example, a subject having their brain activity monitored by the brain monitoring system 120 is given assistance to fall asleep by the sleep regulatory unit 150. The sensory and monitoring unit 140 detects when the subject has fallen asleep and sends this information to the processing system 110. Once the neuronal networks associated with a sleep state have been fully established in the subject, the sleep regulatory unit wakes the subject up. The sensory and monitoring unit detects when the subject has woken up. This process continues, with the brain monitoring system monitoring the brain activity of the subject throughout the process, until a predefined number of instances of falling asleep have been recorded. Once the process is completed, the processing system 110 processes the brain activity data 125 obtained by the brain monitoring system and obtains a value for one or more transition parameters for each recorded instance of falling asleep. The processing system determines which of a plurality of groups of subjects the subject most closely resembles based on the obtained values, and outputs this information to the display device 130.

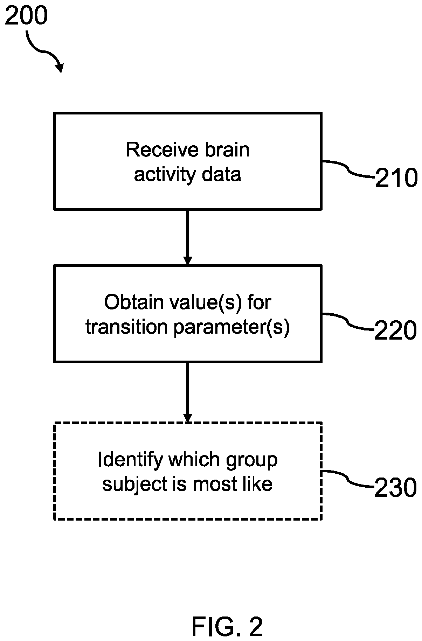

Fig. 2 illustrates a computer-implemented method 200 for analyzing the brain activity of a subject during a transition between brain states, as implemented by a system and a computer program product according to an embodiment of the invention.

The method 200 begins with step 210, in which brain activity data is received from a brain monitoring system. The brain activity data comprises data obtained during a transition of the subject from a first brain state of the subject to a second brain state, where at least one of the first brain state and/or the second brain state is a sleep state.

At step 220, the brain activity data is processed to obtain a value for one or more transition parameters of the brain activity data. A transition parameter is a parameter representative of the transition.

The method 200 may also comprise a step 230, in which the value(s) of the one or more transition parameters of the brain activity data and corresponding values of the one or more transition parameters for a plurality of groups of subjects are processed to identify which of the plurality of groups the subject most closely resembles.

It will be understood that the disclosed methods are computer-implemented methods. As such, there is proposed a concept of a computer program comprising computer program code for implementing any described method when said program is run on a processing system.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A processing system (110) for analyzing the brain activity of a subject during a transition between brain states, the processing system being configured to:
- receive, from a brain monitoring system (120) for monitoring brain activity, brain activity data (125) of the subject obtained during a transition of the subject from a first brain state to a second brain state of the subject, wherein at least one of the first brain state and/or the second brain state is a sleep state;
- receive, from a sensory and monitoring unit (140) adapted to detect a transition of the subject from the first brain state to the second brain state of the subject, information corresponding to the detected transition;
- process the brain activity data and the information from the sensory and monitoring unit to obtain a value for one or more transition parameters of the brain activity data, a transition parameter being a parameter representative of the transition, wherein the one or more transition parameters comprise at least one of:
a transition timing, wherein a transition timing is a length of time between a time at which a transition is detected in the subject by the sensory and monitoring unit and a time at which changes in neuronal networks, identifiable in the subject's brain activity data, responsive to the change in sleep state are first detected;
a transition duration, wherein a transition duration is a duration from a moment at which a neuronal network associated with the first brain state of the subject starts to become weaker to a moment at which a neuronal network associated with the second brain state becomes fully established;
a transition stability, wherein a transition stability is a number of transitions between neuronal networks in the brain activity data from a moment at which activity in neuronal networks associated with the first brain state starts to become weaker to a moment at which a network associated with the second brain state becomes fully established; and/or
a frequency of transition, wherein a frequency of transition is a measure of the number of times a transition from the first brain state to the second brain state occurs in a set time period; and **characterized in that** the processing system is further configured to:
- process the values of the one or more transition parameters of the brain activity data (125) and corresponding values of the one or more transition parameters for a plurality of groups of subjects to identify which of the plurality of groups the subject most closely resembles, wherein the plurality of groups of subjects comprises at least a first group and a second group, wherein the first group comprises healthy subjects and the second group comprises subjects having a mental disorder.

2. The processing system (110) of claim 1, wherein the step of processing the values of the one or more transition parameters of the brain activity data (125) and corresponding values of the one or more transition parameters for a plurality of groups of subjects further uses one or more characteristics of the subject to identify which of the plurality of groups the subject most closely resembles.

3. The processing system (110) of claim 1 or 2, wherein the step of processing the values of the one or more transition parameters of the brain activity data (125) and corresponding values of the one or more transition parameters for a plurality of groups of subjects to identify which of the plurality of groups the subject most closely resembles comprises:
inputting the brain activity data and/or the values of the one or more transition parameters into an artificial neural network (115).

4. The processing system (110) of claim 3, wherein the artificial neural network (115) has been trained using a training algorithm configured to receive an array of training inputs and known outputs, wherein the training inputs comprise brain activity data and/or values of one or more transition parameters during transitions from a first brain state to a second brain state, and the known outputs comprise a determination of which of a plurality of groups of subjects the brain activity data belongs to.

5. The processing system (110) of any of claims 1 to 4, wherein the transition is one of:
a transition from a wakeful state to a sleep state;
a transition from a sleep state to a wakeful state; or
a transition from a first sleep state to a second, different sleep state.

6. The processing system (110) of any of claims 1 to 5, wherein the one or more transition parameters further comprise the one or more networks active during the transition.

7. The processing system (110) of any of claims 1 to 6, wherein the processing system is configured to continue receiving brain activity data (125) of the subject until a predefined number of transitions have been recorded, and to obtain a value for one or more transition parameters of the brain activity data for each detected transition.

8. A system (100) comprising:
- a sensory and monitoring unit (140) adapted to detect a transition of the subject from a first brain state to a second brain state of the subject, wherein at least one of the first brain state and/or the second brain state is a sleep state; and
- the processing system (110) of any of claims 1 to 7.

9. The system (100) of claim 8, wherein the sensory and monitoring unit (140) is adapted to detect a transition based on at least one of: brain activity information, cardiorespiratory information, cardioballistography information, respiration rate, behavioral information and/or information corresponding to the subject's performance on a repetitive task.

10. The system (100) of claims 8 or 9, wherein the system further comprises a sleep regulatory unit (150) adapted to induce a change in brain state of the subject.

11. The system (100) of claim 10, wherein the sleep regulatory unit (150) is adapted to alternately induce sleep in the subject and wake the subject from sleep for a predetermined number of wake/sleep cycles.

12. A computer program product comprising computer program code which, when executed on a computing device having a processing system, cause the processing system to perform a computer-implemented method (200) for analyzing the brain activity of a subject during a transition between brain states, the computer-implemented method comprising:
- receiving, from a brain monitoring system (120) for monitoring brain activity, brain activity data (125) of the subject obtained during a transition of the subject from a first brain state to a second brain state of the subject, wherein at least one of the first brain state and/or the second brain state is a sleep state;
- receiving, from a sensory and monitoring unit (140) adapted to detect a transition of the subject from the first brain state to the second brain state of the subject, information corresponding to the detected transition;
- processing the brain activity data and the information from the sensory and monitoring unit to obtain a value for one or more transition parameters of the brain activity data, a transition parameter being a parameter representative of the transition, wherein the one or more transition parameters comprise at least one of:
a transition timing, wherein a transition timing is a length of time between a time at which a transition is detected in the subject and a time at which changes in neuronal networks, identifiable in the subject's brain activity data, responsive to the change in sleep state are first detected;
a transition duration, wherein a transition duration is a duration from a moment at which a neuronal network associated with the first brain state of the subject starts to become weaker to a moment at which a neuronal network associated with the second brain state becomes fully established;
a transition stability, wherein a transition stability is a number of transitions between neuronal networks in the brain activity data from a moment at which activity in neuronal networks associated with the first brain state starts to become weaker to a moment at which a network associated with the second brain state becomes fully established; and/or
a frequency of transition, wherein a frequency of transition is a measure of the number of times a transition from the first brain state to the second brain state occurs in a set time period; and
- processing the values of the one or more transition parameters of the brain activity data (125) and corresponding values of the one or more transition parameters for a plurality of groups of subjects to identify which of the plurality of groups the subject most closely resembles, wherein the plurality of groups of subjects comprises at least a first group and a second group, wherein the first group comprises healthy subjects and the second group comprises subjects having a mental disorder.

## Patentansprüche

1. Verarbeitungssystem (110) zum Analysieren der Gehirnaktivität eines Subjekts während eines Übergangs zwischen Gehirnzuständen, wobei das Verarbeitungssystem so konfiguriert ist, dass es:
- von einem Gehirnüberwachungssystem (120) zur Überwachung von Gehirnaktivität Gehirnaktivitätsdaten (125) des Subjekts, die während eines Übergangs des Subjekts von einem ersten Gehirnzustand in einen zweiten Gehirnzustand des Subjekts erhalten werden, empfängt, wobei mindestens einer des ersten Gehirnzustands und/oder des zweiten Gehirnzustands ein Schlafzustand ist;
- von einer Sensor- und Überwachungseinheit (140), die dazu ausgelegt ist, einen Übergang des Subjekts vom ersten Gehirnzustand in den zweiten Gehirnzustand des Subjekts zu erkennen, Informationen empfängt, die dem erkannten Übergang entsprechen;
- die Gehirnaktivitätsdaten und die Informationen von der Sensor- und Überwachungseinheit verarbeitet, um einen Wert für einen oder mehrere Übergangsparameter der Gehirnaktivitätsdaten zu erhalten, wobei ein Übergangsparameter ein Parameter ist, der für den Übergang repräsentativ ist, wobei der eine oder die mehreren Übergangsparameter mindestens eines umfassen von:
- einer Übergangszeit, wobei eine Übergangszeit eine Zeitlänge zwischen einem Zeitpunkt, zu dem ein Übergang bei dem Subjekt durch die Sensor- und Überwachungseinheit erkannt wird, und einem Zeitpunkt ist, zu dem Änderungen in neuronalen Netzen, die in den Gehirnaktivitätsdaten des Subjekts in Reaktion auf die Änderung des Schlafzustands identifiziert werden können, erstmals erkannt werden;
einer Übergangsdauer, wobei eine Übergangsdauer eine Dauer von einem Moment, an dem ein neuronales Netz, das dem ersten Gehirnzustand des Subjekts zugeordnet ist, schwächer zu werden beginnt, bis zu einem Moment ist, an dem ein neuronales Netz, das dem zweiten Gehirnzustand zugeordnet ist, vollständig etabliert ist;
einer Übergangsstabilität, wobei eine Übergangsstabilität eine Anzahl von Übergängen zwischen neuronalen Netzen in den Gehirnaktivitätsdaten von einem Moment, an dem Aktivität in neuronalen Netzen, die dem ersten Gehirnzustand zugeordnet sind, schwächer zu werden beginnt, bis zu einem Moment ist, an dem ein Netz, das dem zweiten Gehirnzustand zugeordnet ist, vollständig etabliert ist; und/oder
einer Übergangshäufigkeit, wobei eine Übergangshäufigkeit ein Maß dafür ist, wie oft ein Übergang vom ersten Gehirnzustand in den zweiten Gehirnzustand in einem festgelegten Zeitraum auftritt; und **dadurch gekennzeichnet, dass** das Verarbeitungssystem weiter so konfiguriert ist, dass es:
- die Werte des einen oder der mehreren Übergangsparameter der Gehirnaktivitätsdaten (125) und entsprechende Werte des einen oder der mehreren Übergangsparameter für eine Vielzahl von Subjektgruppen verarbeitet, um zu identifizieren, welcher der Vielzahl von Gruppen das Subjekt am meisten ähnelt, wobei die Vielzahl von Subjektgruppen mindestens eine erste Gruppe und eine zweite Gruppe umfasst, wobei die erste Gruppe gesunde Subjekte umfasst, und die zweite Gruppe Subjekte umfasst, die eine psychische Störung aufweisen.

2. Verarbeitungssystem (110) nach Anspruch 1, wobei der Schritt des Verarbeitens der Werte des einen oder der mehreren Übergangsparameter der Gehirnaktivitätsdaten (125) und entsprechender Werte des einen oder der mehreren Übergangsparameter für eine Vielzahl von Subjektgruppen weiter eine oder mehrere Kennzeichen des Subjekts verwendet, um zu identifizieren, welcher der Vielzahl von Gruppen das Subjekt am meisten ähnelt.

3. Verarbeitungssystem (110) nach Anspruch 1 oder 2, wobei der Schritt des Verarbeitens der Werte des einen oder der mehreren Übergangsparameter der Gehirnaktivitätsdaten (125) und entsprechender Werte des einen oder der mehreren Übergangsparameter für eine Vielzahl von Subjektgruppen, um zu identifizieren, welcher der Vielzahl von Gruppen das Subjekt am meisten ähnelt, umfasst:
Eingeben der Gehirnaktivitätsdaten und/oder der Werte des einen oder der mehreren Übergangsparameter in ein künstliches neuronales Netz (115).

4. Verarbeitungssystem (110) nach Anspruch 3, wobei das künstliche neuronale Netz (115) unter Verwendung eines Trainingsalgorithmus, der so konfiguriert ist, dass er eine Reihe von Trainingseingaben und bekannten Ausgaben empfängt, trainiert wurde, wobei die Trainingseingaben Gehirnaktivitätsdaten und/oder Werte eines oder mehrerer Übergangsparameter während Übergängen von einem ersten Gehirnzustand in einen zweiten Gehirnzustand umfassen, und die bekannten Ausgaben eine Bestimmung desbezüglich umfassen, zu welcher einer Vielzahl von Subjektgruppen die Gehirnaktivitätsdaten gehören.

5. Verarbeitungssystem (110) nach einem der Ansprüche 1 bis 4, wobei der Übergang einer ist von:
einem Übergang von einem Wachzustand in einen Schlafzustand;
einem Übergang von einem Schlafzustand in einen Wachzustand; oder
einem Übergang von einem ersten Schlafzustand in einen zweiten, anderen Schlafzustand.

6. Verarbeitungssystem (110) nach einem der Ansprüche 1 bis 5, wobei der eine oder die mehreren Übergangsparameter weiter das eine oder die mehreren Netze, die während des Übergangs aktiv sind, umfassen.

7. Verarbeitungssystem (110) nach einem der Ansprüche 1 bis 6, wobei das Verarbeitungssystem so konfiguriert ist, dass es den Empfang von Gehirnaktivitätsdaten (125) des Subjekts fortsetzt, bis eine vordefinierte Anzahl von Übergängen aufgezeichnet wurde, und für jeden erkannten Übergang einen Wert für einen oder mehrere Übergangsparameter der Gehirnaktivitätsdaten erhält.

8. System (100), umfassend:
- eine Sensor- und Überwachungseinheit (140), die dazu ausgelegt ist, einen Übergang des Subjekts von einem ersten Gehirnzustand in einen zweiten Gehirnzustand des Subjekts zu erkennen, wobei mindestens einer des ersten Gehirnzustands und/oder des zweiten Gehirnzustands ein Schlafzustand ist; und
- das Verarbeitungssystem (110) nach einem der Ansprüche 1 bis 7.

9. System (100) nach Anspruch 8, wobei die Sensor- und Überwachungseinheit (140) dazu ausgelegt ist, einen Übergang auf Basis mindestens eines zu erkennen von: Gehirnaktivitätsinformationen, kardiorespiratorischen Informationen, Kardioballistographie-Informationen, Atemfrequenz, Verhaltensinformationen und/oder Informationen, die der Leistung des Subjekts bei einer sich wiederholenden Aufgabe entsprechen.

10. System (100) nach den Ansprüchen 8 oder 9, wobei das System weiter eine Schlafregulierungseinheit (150) umfasst, die dazu ausgelegt ist, eine Änderung des Gehirnzustands des Subjekts zu induzieren.

11. System (100) nach Anspruch 10, wobei die Schlafregulierungseinheit (150) dazu ausgelegt ist, über eine vorbestimmte Anzahl von Wach-/Schlafzyklen abwechselnd Schlaf des Subjekts zu induzieren und das Subjekt aus dem Schlaf zu wecken.

12. Computerprogrammprodukt, das Computerprogrammcode umfasst, der, wenn er auf einer Rechenvorrichtung, die ein Verarbeitungssystem aufweist, ausgeführt wird, das Verarbeitungssystem dazu bringt, ein computerimplementiertes Verfahren (200) zum Analysieren der Gehirnaktivität eines Subjekts während eines Übergangs zwischen Gehirnzuständen durchzuführen, wobei das computerimplementierte Verfahren umfasst:
- Empfangen, von einem Gehirnüberwachungssystem (120) zur Überwachung von Gehirnaktivität, von Gehirnaktivitätsdaten (125) des Subjekts, die während eines Übergangs des Subjekts von einem ersten Gehirnzustand in einen zweiten Gehirnzustand des Subjekts erhalten werden, wobei mindestens einer des ersten Gehirnzustands und/oder des zweiten Gehirnzustands ein Schlafzustand ist;
- Empfangen, von einer Sensor- und Überwachungseinheit (140), die dazu ausgelegt ist, einen Übergang des Subjekts vom ersten Gehirnzustand in den zweiten Gehirnzustand des Subjekts zu erkennen, von Informationen, die dem erkannten Übergang entsprechen;
- Verarbeiten der Gehirnaktivitätsdaten und der Informationen von der Sensor- und Überwachungseinheit, um einen Wert für einen oder mehrere Übergangsparameter der Gehirnaktivitätsdaten zu erhalten, wobei ein Übergangsparameter ein Parameter ist, der für den Übergang repräsentativ ist, wobei der eine oder die mehreren Übergangsparameter mindestens eines umfassen von:
einer Übergangszeit, wobei eine Übergangszeit eine Zeitlänge zwischen einem Zeitpunkt, zu dem ein Übergang bei dem Subjekt erkannt wird, und einem Zeitpunkt ist, zu dem Änderungen in neuronalen Netzen, die in den Gehirnaktivitätsdaten des Subjekts in Reaktion auf die Änderung des Schlafzustands identifiziert werden können, erstmals erkannt werden;
einer Übergangsdauer, wobei eine Übergangsdauer eine Dauer von einem Moment, an dem ein neuronales Netz, das dem ersten Gehirnzustand des Subjekts zugeordnet ist, schwächer zu werden beginnt, bis zu einem Moment ist, an dem ein neuronales Netz, das dem zweiten Gehirnzustand zugeordnet ist, vollständig etabliert ist;
einer Übergangsstabilität, wobei eine Übergangsstabilität eine Anzahl von Übergängen zwischen neuronalen Netzen in den Gehirnaktivitätsdaten von einem Moment, an dem Aktivität in neuronalen Netzen, die dem ersten Gehirnzustand zugeordnet sind, schwächer zu werden beginnt, bis zu einem Moment ist, an dem ein Netz, das dem zweiten Gehirnzustand zugeordnet ist, vollständig etabliert ist; und/oder
einer Übergangshäufigkeit, wobei eine Übergangshäufigkeit ein Maß dafür ist, wie oft ein Übergang vom ersten Gehirnzustand in den zweiten Gehirnzustand in einem festgelegten Zeitraum auftritt; und
- Verarbeiten der Werte des einen oder der mehreren Übergangsparameter der Gehirnaktivitätsdaten (125) und entsprechender Werte des einen oder der mehreren Übergangsparameter für eine Vielzahl von Subjektgruppen, um zu identifizieren, welcher der Vielzahl von Gruppen das Subjekt am meisten ähnelt, wobei die Vielzahl von Subjektgruppen mindestens eine erste Gruppe und eine zweite Gruppe umfasst, wobei die erste Gruppe gesunde Subjekte umfasst, und die zweite Gruppe Subjekte umfasst, die eine psychische Störung aufweisen.

## Revendications

1. Système de traitement (110) pour analyser l'activité cérébrale d'un sujet durant une transition entre des états cérébraux, le système de traitement étant configuré pour :
- recevoir, en provenance d'un système de surveillance cérébrale (120) destiné à surveiller l'activité cérébrale, des données d'activité cérébrale (125) du sujet obtenues durant une transition du sujet d'un premier état cérébral à un second état cérébral du sujet, dans lequel au moins un du premier état cérébral et/ou du second état cérébral est un état de sommeil ;
- recevoir, en provenance d'une unité sensorielle et de surveillance (140) adaptée pour détecter une transition du sujet du premier état cérébral au second état cérébral du sujet, des informations correspondant à la transition détectée ;
- traiter les données d'activité cérébrale et les informations provenant de l'unité sensorielle et de surveillance pour obtenir une valeur pour un ou plusieurs paramètres de transition des données d'activité cérébrale, un paramètre de transition étant un paramètre représentatif de la transition, dans lequel les un ou plusieurs paramètres de transition comprennent au moins un de :
un moment de transition, dans lequel un moment de transition est un laps de temps entre un instant auquel une transition est détectée chez le sujet par l'unité sensorielle et de surveillance et un instant auquel des changements dans les réseaux neuronaux, identifiables dans les données d'activité cérébrale du sujet, en réponse au changement d'état de sommeil, sont détectés pour la première fois ;
une durée de transition, dans lequel une durée de transition est une durée allant d'un moment auquel un réseau neuronal associé au premier état cérébral du sujet commence à s'affaiblir jusqu'à un moment auquel un réseau neuronal associé au second état cérébral est complètement établi ;
une stabilité de transition, dans lequel une stabilité de transition est un nombre de transitions entre des réseaux neuronaux dans les données d'activité cérébrale depuis un moment auquel l'activité dans les réseaux neuronaux associés au premier état cérébral commence à s'affaiblir jusqu'à un moment auquel un réseau associé au second état cérébral est complètement établi ; et/ou
une fréquence de transition, dans lequel une fréquence de transition est une mesure du nombre de fois qu'une transition du premier état cérébral au second état cérébral se produit dans une période de temps définie ; et **caractérisé en ce que** le système de traitement est en outre configuré pour :
- traiter les valeurs des un ou plusieurs paramètres de transition des données d'activité cérébrale (125) et des valeurs correspondantes des un ou plusieurs paramètres de transition pour une pluralité de groupes de sujets afin d'identifier à quel groupe de la pluralité de groupes le sujet ressemble le plus, dans lequel la pluralité de groupes de sujets comprennent au moins un premier groupe et un second groupe, dans lequel le premier groupe comprend des sujets sains et le second groupe comprend des sujets présentant un trouble mental.

2. Système de traitement (110) selon la revendication 1, dans lequel l'étape consistant à traiter les valeurs des un ou plusieurs paramètres de transition des données d'activité cérébrale (125) et des valeurs correspondantes des un ou plusieurs paramètres de transition pour une pluralité de groupes de sujets utilise en outre une ou plusieurs caractéristiques du sujet pour identifier à quel groupe de la pluralité de groupes le sujet ressemble le plus.

3. Système de traitement (110) selon la revendication 1 ou 2, dans lequel l'étape consistant à traiter les valeurs des un ou plusieurs paramètres de transition des données d'activité cérébrale (125) et des valeurs correspondantes des un ou plusieurs paramètres de transition pour une pluralité de groupes de sujets pour identifier à quel groupe de la pluralité de groupes le sujet ressemble le plus consiste à :
entrer les données d'activité cérébrale et/ou les valeurs des un ou plusieurs paramètres de transition dans un réseau neuronal artificiel (115).

4. Système de traitement (110) selon la revendication 3, dans lequel le réseau neuronal artificiel (115) a été formé à l'aide d'un algorithme de formation configuré pour recevoir un ensemble d'entrées de formation et de sorties connues, dans lequel les entrées de formation comprennent des données d'activité cérébrale et/ou des valeurs d'un ou de plusieurs paramètres de transition durant des transitions d'un premier état cérébral à un second état cérébral, et les sorties connues comprennent une détermination du groupe d'une pluralité de groupes de sujets auquel appartiennent les données d'activité cérébrale.

5. Système de traitement (110) selon l'une quelconque des revendications 1 à 4, dans lequel la transition est l'une parmi :
une transition d'un état d'éveil à un état de sommeil ;
une transition d'un état de sommeil à un état d'éveil ; ou
une transition d'un premier état de sommeil à un second état de sommeil différent.

6. Système de traitement (110) selon l'une quelconque des revendications 1 à 5, dans lequel les un ou plusieurs paramètres de transition comprennent en outre les un ou plusieurs réseaux actifs durant la transition.

7. Système de traitement (110) selon l'une quelconque des revendications 1 à 6, dans lequel le système de traitement est configuré pour continuer de recevoir des données d'activité cérébrale (125) du sujet jusqu'à ce qu'un nombre prédéfini de transitions aient été enregistrées, et pour obtenir une valeur pour un ou plusieurs paramètres de transition des données d'activité cérébrale pour chaque transition détectée.

8. Système (100) comprenant :
- une unité sensorielle et de surveillance (140) adaptée pour détecter une transition du sujet d'un premier état cérébral à un second état cérébral du sujet, dans lequel au moins un du premier état cérébral et/ou du second état cérébral est un état de sommeil ; et
- le système de traitement (110) selon l'une quelconque des revendications 1 à 7.

9. Système (100) selon la revendication 8, dans lequel l'unité sensorielle et de surveillance (140) est adaptée pour détecter une transition sur la base d'au moins un élément parmi : des informations d'activité cérébrale, des informations cardiorespiratoires, des informations de cardioballistographie, une fréquence respiratoire, des informations de comportement et/ou des informations correspondant à la réalisation, par le sujet, d'une tâche répétitive.

10. Système (100) selon les revendications 8 ou 9, dans lequel le système comprend en outre une unité de régulation de sommeil (150) adaptée pour causer un changement de l'état cérébral du sujet.

11. Système (100) selon la revendication 10, dans lequel l'unité de régulation de sommeil (150) est adaptée pour causer en alternance le sommeil chez le sujet et l'éveil du sujet du sommeil pendant un nombre prédéterminé de cycles d'éveil/de sommeil.

12. Produit de programme informatique comprenant un code de programme informatique qui, lorsqu'il est exécuté sur un dispositif informatique présentant un système de traitement, amène le système de traitement à réaliser un procédé mis en œuvre par ordinateur (200) pour analyser l'activité cérébrale d'un sujet durant une transition entre des états cérébraux, le procédé mis en œuvre par ordinateur consistant à :
- recevoir, en provenance d'un système de surveillance cérébrale (120) destiné à surveiller l'activité cérébrale, des données d'activité cérébrale (125) du sujet obtenues durant une transition du sujet d'un premier état cérébral à un second état cérébral du sujet, dans lequel au moins un du premier état cérébral et/ou du second état cérébral est un état de sommeil ;
- recevoir, en provenance d'une unité sensorielle et de surveillance (140) adaptée pour détecter une transition du sujet du premier état cérébral au second état cérébral du sujet, des informations correspondant à la transition détectée ;
- traiter les données d'activité cérébrale et les informations provenant de l'unité sensorielle et de surveillance pour obtenir une valeur pour un ou plusieurs paramètres de transition des données d'activité cérébrale, un paramètre de transition étant un paramètre représentatif de la transition, dans lequel les un ou plusieurs paramètres de transition comprennent au moins un de :
un moment de transition, dans lequel un moment de transition est un laps de temps entre un instant auquel une transition est détectée chez le sujet et un instant auquel des changements dans les réseaux neuronaux, identifiables dans les données d'activité cérébrale du sujet, en réponse au changement d'état de sommeil, sont détectés pour la première fois ;
une durée de transition, dans lequel une durée de transition est une durée allant d'un moment auquel un réseau neuronal associé au premier état cérébral du sujet commence à s'affaiblir jusqu'à un moment auquel un réseau neuronal associé au second état cérébral est complètement établi ;
une stabilité de transition, dans lequel une stabilité de transition est un nombre de transitions entre des réseaux neuronaux dans les données d'activité cérébrale depuis un moment auquel l'activité dans les réseaux neuronaux associés au premier état cérébral commence à s'affaiblir jusqu'à un moment auquel un réseau associé au second état cérébral est complètement établi ; et/ou
une fréquence de transition, dans lequel une fréquence de transition est une mesure du nombre de fois qu'une transition du premier état cérébral au second état cérébral se produit dans une période de temps définie ; et
- traiter les valeurs des un ou plusieurs paramètres de transition des données d'activité cérébrale (125) et des valeurs correspondantes des un ou plusieurs paramètres de transition pour une pluralité de groupes de sujets afin d'identifier à quel groupe de la pluralité de groupes le sujet ressemble le plus, dans lequel la pluralité de groupes de sujets comprennent au moins un premier groupe et un second groupe, dans lequel le premier groupe comprend des sujets sains et le second groupe comprend des sujets présentant un trouble mental.
